# EUROPÄISCHE PATENTANMELDUNG

(11) **EP 1 974 755 A1**
(43) Veröffentlichungstag der Anmeldung: **01.10.2008**
(21) Anmeldenummer: 07005471.3
(22) Anmeldetag: 16.03.2007
(51) Int. Cl.: A61L 29/14

(54) **Katheter mit sich ändernden Materialeigenschaften**

(71) Anmelder: BrainLAB AG, 85622 Feldkirchen (DE)
(72) Erfinder: Mittermeyer, Stephan, 84036 Landshut (DE); Hartlep, Andreas, 83629 Weyarn (DE)
(74) Vertreter: Schwabe - Sandmair - Marx

(57) **Zusammenfassung**

Die Erfindung betrifft einen Katheter (1) zur Verabreichung einer Substanz in ein Körpergewebe, insbesondere in Gehirnstrukturen, mit einem länglichen Katheterkörper (5), der ein Lumen (2) umgibt, wobei der Katheterkörper (5) über mindestens einen Teil seiner Länge ein Material (3) aufweist, dessen Steifigkeit sich aufgrund von geänderten Umgebungsbedingungen in der Verabreichungsumgebung ändert.

## Beschreibung

Die vorliegende Erfindung betrifft einen Katheter zur Verabreichung einer Substanz in ein Körpergewebe, insbesondere in Gehirnstrukturen. Speziell beschäftigt sich die Erfindung mit Kathetern zur Platzierung in festem Gewebe für die konvektiönsunterstützte Verabreichung von Substanzen. Bei der genannten konvekionsunterstützten Verabreichung von Substanzen wird eine Substanz, beispielsweise ein Medikament, mit Hilfe eines positiven Druckgradienten in ein Gewebe eingebracht.

Damit eine zuverlässige und vorhersagbare Verteilung der Substanz auch über längere Zeit, beispielsweise über mehrere Tage, erfolgen kann, muss der Katheter flexibel sein, wenn er einmal gesetzt ist, um Bewegungen der Verabreichungsumgebung, beispielsweise Bewegungen des Gehirns, mit ausführen zu können und eine homogene Schnittstelle zwischen der äußeren Katheterfläche und dem Gewebe sicherzustellen. Im eingesetzten Zustand zeigen flexible Katheter außerdem einen geringeren Rückfluss als starre Katheter, weil sie sich den Bewegungen, insbesondere Gehirnbewegungen (brain shift), anpassen.

Andererseits sollte der Katheter während des Setzvorgangs so starr wie möglich sein, damit ein genaues stereotaktisches Einsetzen, d.h. eine genaue Positionierung erzielt werden kann. Herkömmlicherweise werden deshalb flexible Katheter mit Hilfe eines Stiletts eingesetzt, das aus einem starren Material hergestellt ist, beispielsweise aus Metall. Die Kombination ergibt einen Katheter, der stereotaktisch exakt entlang einer gewählten Trajektorie gesetzt werden kann; nach dem Setzen wird das Stilett entfernt und der Katheter wird an der Kopfhaut befestigt. Nachteilig wirkt sich hier einerseits aus, dass ein mehrteiliger Instrumentensatz bereitgestellt werden muss, andererseits - und wichtiger - besteht eine hohe Wahrscheinlichkeit, dass beim Entnehmen des Stiletts Luft in das Lumen des Katheters eindringt. Diese Luft wird durch das Einbringen der flüssigen Substanz durch den Katheter auch in das Gewebe eingebracht, was in unzuverlässigen und unvorhersagbaren Fluid-Verteilungsmustern resultiert. Wenn nämlich im Katheter vorhandene Luftblasen in das Gewebe weitergeführt werden, entstehen Druckspitzen während der Infusion die aus der Kompressibilität der Luft in der Infusionsleitung (Lumen) resultieren. Die Luftblasen sammeln sich im Gewebe und/oder entlang der Strömungsbahnen im Gewebe oder bauen selbst neue Strömungsbahnen auf. Diese Störungen können den Rückfluss des Fluids entlang der äußeren Oberfläche des Katheters verstärken, was in einer uneffizienten und unverhersagbaren Fluidverteilung resultiert. Gerade in letzter Zeit ist die Möglichkeit, die Verteilung solcher Infusionen vorherzusagen und damit an eine optimierte Verabreichung zu erreichen, in der Technik wahrgenommen werden. Luftansammlungen, wie sie oben beschrieben wurden und die aus der Verwendung von Stiletten resultieren, stören aber solche Vorausberechnungen massiv.

Aus der US 7,066,931 B2 ist ein Heizkatheter bekannt, der eine variable Steifigkeit aufweist. Um bestimmte Bereiche des Katheters flexibler zu machen, wird vorgeschlagen, in diesen Bereichen Öffnungen, nämlich beispielsweise Einschnitte, Schlitze, Kanäle, Nuten oder Löcher in das Kathetermaterial einzubringen.

Es ist die Aufgabe der vorliegenden Erfindung, einen Katheter zur Verabreichung einer Substanz in ein Körpergewebe bereitzustellen, der sowohl den Anforderungen an die hohe Steifigkeit beim Setzen als auch den Anforderungen an die Flexibilität während seiner Verbleibzeit im Gewebe genügt.

Diese Aufgabe wird durch einen Katheter gemäß dem Patentanspruch 1 gelöst. Die Unteransprüche definieren spezielle Ausführungsformen der vorliegenden Erfindung.

Der erfindungsgemäße, speziell stilettlos setzbare, Katheter zur Verabreichung einer Substanz in ein Körpergewebe, insbesondere in Gehirnstrukturen, weist einen länglichen Katheterkörper auf, der ein Lumen umgibt. Auf mindestens einem Teil seiner Länge umfasst der Katheterkörper ein Material, dessen Steifigkeit sich aufgrund von geänderten Umgebungsbedingungen in der Verabreichungsumgebung ändert.

Mit anderen Worten offenbart die vorliegende Erfindung einen Katheter der sich ab seine Umgebungsbedingungen in gewünschter vorhersagbarer Weise anpasst. Die Erfindung macht von der Tatsache Gebrauch, dass der Katheter ohnehin beim Setzen in eine veränderte Umgebung eintritt, und sie nutzt diese Änderung der Verabreichungsumgebung aus um die Steifigkeiteigenschaften des Katheters zu ändern und den Notwendigkeiten anzupassen. Damit lässt sich sowohl das Setzen des Katheters als auch der Verbleibzustand im Patientengewebe optimieren.

Wenn, wie bei einer Ausführungsform vorgesehen, die Steifigkeit des Materials sich bei Umgebungsbedingungen in der Verabreichungsumgebung verringert, lässt sich erreichen, dass der Katheter beim Einsetzen ohne ein Stilett starr genug ist, um einer vorgesehenen Trajektorie folgen zu können, wobei andererseits durch die Steifigkeitsverringerung nach dem Setzen ein ausreichend flexibler Zustand erreicht wird, um Bewegungen des Gewebes mitzumachen und eine voraussagbare und zuverlässige Medikamentenverabreichung zu garantieren.

Ein weiterer Vorteil des erfindungsgemäßen, stilettlos setzbaren Katheters liegt darin, dass kleinere Katheterdurchmesser realisiert werden können. Dies unterstützt nicht nur die Verringerung von Gewebe-Traumata sondern hilft auch dabei, den Rückfluss von Fluid entlang des Katheters zu verkleinern. Es ist nämlich wissenschaftlich nachgewiesen worden, dass der Rückfluss sich mit geringeren Katheterdurchmessern ebenfalls verringert.

Ein sehr wesentlicher Vorteil der vorliegenden Erfindung liegt in der Tatsache begründet, dass der Katheter stilettlos gesetzt werden kann. Ohne die Notwendigkeit des Einsetzens eines Stiletts kann der Katheter vorab mit dem Infusionsfluid befüllt werden; dies wird als "Priming" bezeichnet. Ein solchermaßen geprimter Katheter hat natürlicherweise nicht den Nachteil, dass durch nachfließendes Fluid Luft (Luftbläschen) in das Gewebe eingebracht wird, und die Fluidverteilung wird zuverlässiger und vorhersagbarer.

Das Material, welches seine Steifigkeit ändert, kann ein gegenüber physikalischen oder chemischen Einflussfaktoren steifigkeitsensitives Material sein. Insbesondere ist das Material eines der folgenden Materialien oder eine Kombination aus den folgenden Materialien:
- ein für elektrische Spannungs- und/oder Stromänderungen sensitives Material;
- ein für magnetische Feldänderungen sensitives Material;
- ein ph-Wert-sensitives Material;
- ein temperatursensitives Material;
- ein für eine Wasserkonzentration sensitives Material;
- ein für eine Ionenkonzentration sensitives Material;
- ein für eine Konzentration einer chemischen Substanz oder Verbindung sensitives Material;
- ein für eine körpereigene Umgebungsbedingung in der Verabreichungsumgebung sensitives Material;
- ein für eine Eigenschaft der zu verabreichende Substanz sensitives Material.

Die Auswahl der Mittel für die jeweilige Änderung der Steifigkeit kann je nach dem am Einsatzort bzw. Verabreichungsort vorliegenden Geräteausstattungen bzw. Umgebungsbedingungen in geeigneter Weise getroffen werden. Es wird erfindungsgemäß die Möglichkeit geschaffen, die körperlichen Eigenschaften des entsprechenden Materials zu verändern, insbesondere die physikalischen oder körperlichen Eigenschaften, wobei die Veränderung durch einen vorhersehbaren oder steuerbaren Einfluss aus der direkten Umgebung ausgelöst wird. Ein Beispiel für Steuerung der Steifigkeit durch eine Wasserkonzentration ist die Verwendung von Hydro-Gels, wie z.B. Silikon-Hydro-Gels als oder im Kathetermaterial.

Wenn hierin von Umgebungsbedingungen in der Verabreichungsumgebung gesprochen wird, kann dies mehrere Bedeutungen haben. Einerseits kann die Steifigkeit des Materials durch äußere Einflüsse geändert werden, also durch Einflüsse, die von außerhalb des Katheters auf diesen einwirken. Andererseits besteht grundsätzlich aber auch die Möglichkeit die Steifigkeit des Kathetermaterials durch "innere Einflüsse" zu steuern, beispielsweise durch eine Wirkung, welche die zu verabreichende Substanz auf das Kathetermaterial ausübt, wenn sie mit einer gewissen Durchflussrate im Lumen des Katheters strömt. Auch Kombinationen solcher Beeinflussungen von außen und innen sind möglich. Ein Beispiel wäre hier, dass die äußeren Bedingungen (z.B. Wasserkonzentration oder Ionenkonzentration) in der Verabreichungsumgebung Voraussetzungen für die Steifigkeitsänderung schaffen, diese Steifigkeitsänderung aber erst dann eintritt, wenn auch die Substanz im Lumen strömt und eine zusätzliche Wirkung ausübt bzw. als Katalysator dient. Man könnte auf diese Weise einen Steuerungsmechanismus erhalten, welcher die Steifigkeit erst dann ändert, wenn tatsächlich Substanzen verabreicht werden.

Der Katheter kann gemäß einer Ausführungsform der vorliegenden Erfindung einen integral geformten Katheterkörper aus mehreren Materialien aufweisen, speziell aus einem inneren Kern, welcher das Lumen umschließt, und einem äußeren Mantel, welcher den Kern umgibt. Natürlich kann der Katheter aber auch ein einziges Material umfassen, welches den Bedingungen für die Änderung der Steifigkeit genügt.

Wenn eine Mantel/Kern-Konfiguration gewählt wird, kann der Mantel ein Material umfassen, welches seine Steifigkeit bei sich verändernden äußeren Bedingungen ändert, während der Kern ein Material umfasst, welches seine Steifigkeit nicht ändert und den Mantel von dem Einfluss der zu verabreichenden Substanz isoliert. Es ist beispielsweise in diesem Kontext möglich, die innere Oberfläche der Katheterröhre mit einer Beschichtung zu versehen, die Schutzkomponenten aufweist, z.B. PTFE (Polytetrafluorethylen = Teflon) etc.

Es besteht ferner die Möglichkeit gerade eine umgekehrte Konfiguration zu wählen, bei der ein Kern ein Material umfasst, welches seine Steifigkeit beim Vorhandensein der zu verabreichenden Substanz ändert, während der Mantel ein Material umfasst, welches seine Steifigkeit nicht ändert und den Kern vor dem Einfluss sich verändernder äußerer Bedingungen isoliert. Generell kann der integral geformte Katheterkörper aus einem oder mehreren Materialien gebaut sein, das bzw. die durch eine Kombination der Umgebungsbedingungen in der äußeren Katheterumgebung und im Lumen eine Steifigkeitsänderung erfährt bzw. erfahren.

Die Erfindung wird im Weiteren anhand von Ausführungsbeispielen und unter Bezugnahme auf die beiliegenden Zeichnungen näher erläutert. Sie kann alle hierin beschriebenen Merkmale einzeln sowie in jedweder sinnvollen Kombination umfassen. In den Zeichnungen zeigen:
- Figur 1: einen Querschnitt durch einen erfindungsgemäßen Katheter;
- Figur 2: eine schamatische Darstellung zur Beeinflussung des Kathetermaterials von außen; und
- Figuren 3 bis 4: jeweils Darstellungen für die Katheter-Steifigkeitsänderung bei unterschiedlichen Umgebungsbedingungen.

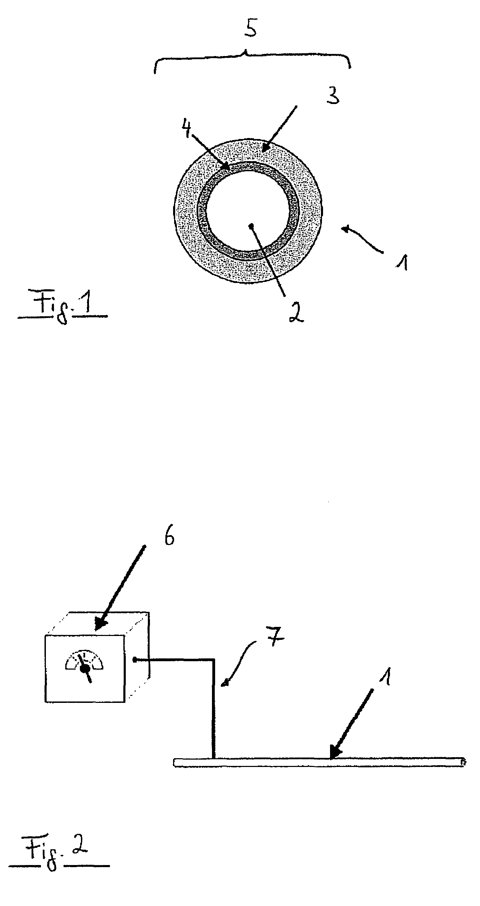

In der Figur 1 ist ein Katheter gemäß der vorliegenden Erfindung im Querschnitt schematisch dargestellt. Der Katheter trägt das Bezugszeichen 1 und er umschließt ein Lumen 2, in dem ein Fluid (z.B. ein Medikament) transportiert wird. Das Lumen 2 ist vom Katheterkörper 5 umgeben, der wiederum einen Kern 4 und einen äußeren Mantel 3 aufweist. Bei einem Ausführungsbeispiel der Erfindung besteht der Mantel 3 aus dem Material, dessen Steifigkeit sich in Anpassung geänderte Umgebungsbedingungen ändert, also flexibler wird, wenn ein äußerer Einfluss wirkt. Das Material 3 kann beispielsweise ein Silikon-Hydro-Gel sein, welches flexibler bzw. weicher wird, wenn es mit Wasser in Berührung kommt.

Der Kern 4 ist beispielsweise eine Teflon-Beschichtung, welche einerseits die Substanz im Lumen 2 vor Einflüssen der äußeren Bedingungen isoliert und andererseits das Material 3 vor Beeinflussungen durch die Substanz im Lumen 2 schützt. Natürlich kann der Katheter 1 in Figur 1 auch einer sein, der durch andere äußere Einflüsse flexibel gemacht wird, als durch eine Wasserkonzentration, und ein Beispiel hierfür ist in Figur 2 dargestellt. Die Figur 2 zeigt den Katheter 1 sowie eine Vorrichtung 6, mit der ein magnetisches Feld oder elektrische Energie (Strom/Spannung) erzeugt werden kann. Die Linie 7 soll schematisch anzeigen, dass die Umgebungsbedingungen, die durch die Vorrichtung 6 erzeugt werden, auf den Katheter 1 wirken. Die Wirkungslinie 7 kann also beispielsweise die Wirkung eines magnetischen oder elektromagnetischen Feldes widerspiegeln, aber auch die Weiterleitung von Strom oder die Erzeugung einer elektrischen Spannung bzw. eines elektrischen Potentials.

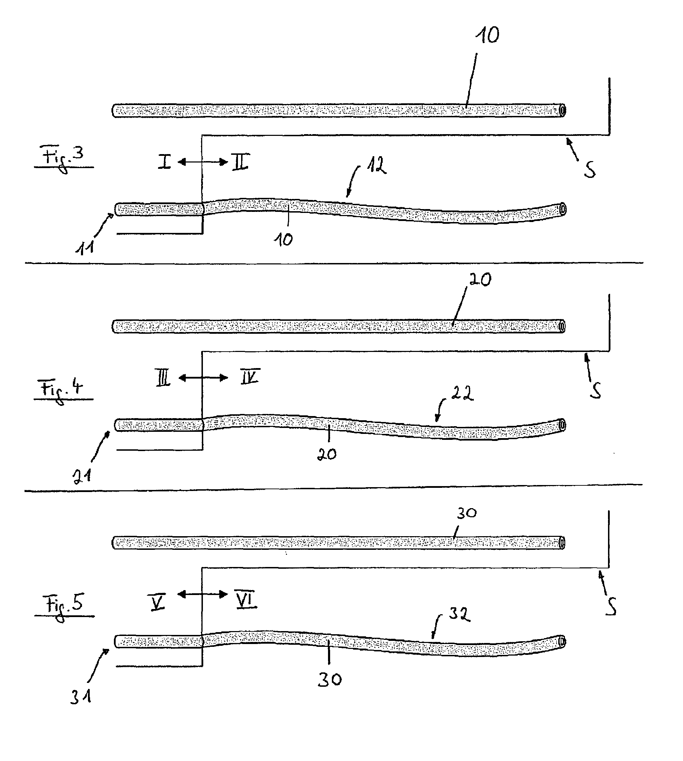

In den Figuren 3 bis 5 sind jeweils Katheter in ihrem Ausgangszustand (oben) und in einem flexibel gemachten Zustand (unten) zu sehen. In jeder der Figuren 3 bis 5 bezeichnet die Linie S eine Separationslinie, welche schematisch andeutet, dass auf den beiden Seiten der Linie unterschiedliche Umgebungsbedingungen herrschen. Die Umgebungsbedingungen sind mit den römischen Zahlen I bis VI bezeichnet.

Die Darstellung der Figur 3 zeigt oben den Katheter 10 in seinem starren Zustand. Auf der Seite I der Separationslinie S herrschen Umgebungsbedingungen, welche das Material des Katheters 10 starr belassen und im Falle der Figur 3 ist eine solche Umgebungsbedingung I ein bestimmter ph-Wert. Auf der anderen Seite II der Separationslinie S herrscht ein hiervon unterschiedlicher ph-Wert, beispielsweise ein etwas niedriger ph-Wert, wie er in Körperflüssigkeiten vorkommen kann. In der unteren Darstellung der Figur 3 ist dann ersichtlich, dass derjenige Teil 11 (proximaler Katheterteil) der sich noch unter den Umgebungsbedingungen I befindet, starr bleibt, und dies ist dadurch dargestellt, dass er gerade verläuft. Auf der Seite, wo die Umgebungsbedingung 11 herrscht, ist der Katheter flexibel geworden, und dies wird dadurch dargestellt, dass sein distaler Abschnitt 12 gebogen gezeichnet ist. Natürlich muss sich der Katheter unter dem Einfluss der Umgebungsbedingung II nicht von selbst biegen, die Biegung dient nur der zeichnerischen Wiedergabe der Flexibilität. Der distale Bereich 12, also beispielsweise der Bereich des Katheters, der im Gehirn eines Patienten verbleibt, kann sich aber aufgrund der gesteigerten Flexibilität biegen, nachdem er im starren Zustand unter Umgebungsbedingungen I eingesetzt wurde.

Die Figuren 4 und 5 entsprechen in ihrer grundsätzlichen Darstellung der Figur 3 und stellen jeweils einen Katheter 20 bzw. 30 dar, der unter den Umgebungsbedingungen III bzw. V steif ist bzw. bleibt, aber unter den Umgebungsbedingungen IV und VI flexibel wird, und zwar zumindest im distalen Bereich 23 oder 32, während der Bereich jenseits der Separationslinie S, nämlich der proximale Abschnitt 21 bzw. 31 starr bleibt. Die Ausführungsformen der Figuren 4 und 5 unterscheiden sich durch jeweils andere Umgebungsbedingungen, so steht beispielsweise die Umgebungsbedingung III für eine bestimmte chemische Umgebung, insbesondere eine Luftumgebung. Die chemische Umgebung II ist dann eine Umgebung, in der beispielsweise eine gewisse Feuchtigkeit herrscht oder spezielle chemische Substanzen präsent sind, wobei diese Einflüsse wiederum darauf hinwirken den distalen Abschnitt 22 flexibler zu machen als er im Ausgangszustand unter den chemischen Bedingungen III war.

Analoges gilt für die Darstellung der Figur 5, wobei auf der mit V bezeichneten Seite der Separationslinie S eine andere magnetische oder elektrische Umgebung herrscht als auf der Seite VI ein Beispiel hierfür ist, dass in der Umgebung V kein Magnetfeld präsent ist, während in der Umgebung VI ein Magnetfeld durch einen entsprechenden Magnetfeldgenerator erzeugt worden ist. Dies bewirkt, dass der distale Teil 32 des Katheters 30 flexibel wird, während der proximale Teil 31 des Katheters 30, der sich außerhalb des Magnetfeldes befindet, starr bleibt.

Unter den Umgebungsbedingungen 1, III und V kann der Katheter als starrer Körper stereotaktisch exakt gesetzt werden. Nach einer gewissen Anpassungszeit ändert sich die Materialsteifigkeit und unter den Umgebungsbedingungen II, IV und VI ist er flexibel genug, um auch bei längerem Verbleiben im Gewebe, z.B. im Gehirngewebe, dessen Bewegungen mitzumachen und dadurch Traumata zu verhindern und eine ortsgenaue Infusion zu gewährleisten.

## Patentansprüche

1. Katheter (1) zur Verabreichung einer Substanz in ein Körpergewebe, insbesondere in Gehirnstrukturen, mit einem länglichen Katheterkörper (5), der ein Lumen (2) umgibt, **dadurch gekennzeichnet, dass** der Katheterkörper (5) über mindestens einen Teil seiner Länge ein Material (3) aufweist, dessen Steifigkeit sich aufgrund von geänderten Umgebungsbedingungen in der Verabreichungsumgebung ändert.

2. Katheter nach Anspruch 1, **dadurch gekennzeichnet, dass** die Steifigkeit des Materials (3) sich bei Umgebungsbedingungen in der Verabreichungsumgebung verringert.

3. Katheter nach Anspruch 1 oder 2, **dadurch gekennzeichnet, dass** das Material (3) ein gegenüber physikalischen oder chemischen Einflussfaktoren steifigkeitssensitives Material ist.

4. Katheter nach Anspruch 3, **dadurch gekennzeichnet, dass** das Material ein für elektrische Spannungs- und/oder Stromänderungen sensitives Material ist.

5. Katheter nach Anspruch 3 oder 4, **dadurch gekennzeichnet, dass** das Material ein für magnetische Feldänderungen sensitives Material ist.

6. Katheter nach einem der Ansprüche 3 bis 5, **dadurch gekennzeichnet, dass** das Material ein pH-Wert-sensitives Material ist.

7. Katheter nach einem der Ansprüche 3 bis 6, **dadurch gekennzeichnet, dass** das Material ein temperatursensitives Material ist.

8. Katheter nach einem der Ansprüche 3 bis 7, **dadurch gekennzeichnet, dass** das Material ein für eine Wasserkonzentration sensitives Material ist.

9. Katheter nach einem der Ansprüche 3 bis 8, **dadurch gekennzeichnet, dass** das Material ein für eine Ionenkonzentration sensitives Material ist.

10. Katheter nach einem der Ansprüche 3 bis 9, **dadurch gekennzeichnet, dass** das Material ein für eine Konzentration einer chemischen Substanz oder Verbindung sensitives Material ist.

11. Katheter nach einem der Ansprüche 3 bis 10, **dadurch gekennzeichnet, dass** das Material ein für eine körpereigene Umgebungsbedingung in der Verabreichungsumgebung sensitives Material ist.

12. Katheter nach einem der Ansprüche 3 bis 11, **dadurch gekennzeichnet, dass** das Material ein für eine Eigenschaft der zu verabreichenden Substanz sensitives Material ist.

13. Katheter nach einem der Ansprüche 1 bis 12, **dadurch gekennzeichnet, dass** er einen integral geformten Katheterkörper (5) aus mehreren Materialen aufweist, speziell aus einem inneren Kern (4), welcher das Lumen (2) umschließt, und einem äußeren Mantel (3), welcher den Kern (4) umgibt.

14. Katheter nach Anspruch 13, **dadurch gekennzeichnet, dass** der Mantel (3) ein Material umfasst, welches seine Steifigkeit bei sich verändernden äußeren Bedingungen ändert, während der Kern (4) ein Material umfasst, welches seine Steifigkeit nicht ändert und den Mantel vor dem Einfluss der zu verabreichenden Substanz isoliert.

15. Katheter nach Anspruch 13, **dadurch gekennzeichnet, dass** der Kern (4) ein Material umfasst, welches seine Steifigkeit beim Vorhandensein der zu verabreichenden Substanz ändert, während der Mantel (3) ein Material umfasst, welches seine Steifigkeit nicht ändert und den Kern vor dem Einfluss sich verändernder äußerer Bedingungen isoliert.

16. Katheter nach einem der Ansprüche 1 bis 12, **dadurch gekennzeichnet, dass** er einen integral geformten Katheterkörper (5) aus einem oder mehreren Materialen aufweist, das bzw. die durch eine Kombination der Umgebungsbedingungen in der äußeren Katheterumgebung und im Lumen eine Steifigkeitsänderung erfährt bzw. erfahren.

## Geänderte Patentansprüche

### Geänderte Patentansprüche gemäss Regel 137(2) EPÜ.

**1.** Katheter (1) zur Verabreichung einer Substanz in ein Körpergewebe, insbesondere in Gehirnstrukturen, mit einem länglichen Katheterkörper (5), der ein Lumen (2) umgibt, wobei der Katheterkörper (5) über mindestens einen Teil seiner Länge ein Material (3) aufweist, dessen Steifigkeit sich aufgrund von geänderten Umgebungsbedingungen in der Verabreichungsumgebung ändert, **dadurch gekennzeichnet, dass** das Material (3) ein gegenüber magnetischen Feldänderungen steifigkeitssensitives Material ist.
